# EUROPEAN PATENT APPLICATION

(11) **EP 1 555 263 A1**
(43) Date of publication of application: **20.07.2005**
(21) Application number: 03751271.2
(22) Date of filing: 17.09.2003
(51) Int. Cl.: C07D 405/12, A61K 31/4525, A61P 25/00, A61P 25/04, A61P 25/22, A61P 25/24, A61P 25/28, A61P 43/00

(54) **PROCESS FOR PRODUCING PAROXETINE HYDROCHLORIDE HYDRATE**

(30) Priority: 19.09.2002 JP 2002273901; 01.10.2002 JP 2002288640
(71) Applicant: Sumitomo Chemical Company, Limited, Tokyo 104-8260 (JP)
(72) Inventor: YAMAZAKI, Shigeya, Sumitomo Chemical Company, Ltd, Osaka-shi, Osaka 555-0021 (JP); YOSHIKAWA, Taichi, Sumitomo Chemical Company, Ltd, Osaka-shi, Osaka 555-0021 (JP)
(74) Representative: Marchant, James Ian
(86) International application number: PCT/JP2003/011806
(87) International publication number: WO 2004/026861

(57) **Abstract**

A process for preparing a paroxetine hydrochloride hydrate, comprising reacting (3S, 4R)-1-tert-butoxycarbonyl-4-(4-fluorophenyl)-3-[(3,4-methylenedioxy)phenoxymethyl]piperidine with hydrogen chloride in the presence of water, and allowing crystals to separate out from the resulting reaction mixture in the presence of water.

## Description

### TECPINICAL FIELD

A paroxetine hydrochloride hydrate [(-)-(3S,4R)-4-(4-fluorophenyl)-3-[(3,4-methylenedioxy)phenoxymethyl]piperidine monohydrochloride hemihydrate] has been known worldwide as an antidepressant for treating and/or preventing a disorder selected from the group consisting of alcoholism, anxiety syndrome, depression, obsessive-compulsive disorder, panic disorder, chronic pain, obesity, senile dementia, migraine, polyphagia, inappetence, social phobia, premenstrual tension syndrome, adolescent depression, trichotillomania, dysthymia and substance abuse. The present invention relates to a novel process for preparing the paroxetine hydrochloride hydrate.

### BACKGROUND ART

Conventionally, as a process for preparing a paroxetine hydrochloride in which (3S,4R)-1-tert-butoxycarbonyl-4-(4-fluorophenyl)-3-[(3,4-methylenedioxy)phenoxymethyl]piperidine (hereinafter referred to as BOC-protected paroxetine) is used as a raw material, a process comprising reacting a BOC-protected paroxetine with hydrogen chloride in isopropanol has been known. This product is a paroxetine hydrochloride anhydrate, as disclosed in European Patent Publication No. 0812827 A1 (corresponding to JP-A-10-291975). On the other hand, a process for preparing the paroxetine hydrochloride hydrate which comprises converting the crystal form of this anhydrate has been disclosed in known publications. According to this process, the conversion of crystal form is carried out by applying pressure, or in the presence of a seed crystal under the circumstances having a high humidity, as disclosed in European Patent No. 223403 B1 (corresponding to JP-B-6-47587) and International Journal of Pharmaceutics, 42(1988), 135-143.

A variety of processes for preparing a paroxetine hydrochloride hydrate have been disclosed in International Journal of Pharmaceutics, 42 (1988), 135-143 and European Patent No. 223403 B1. However, these documents do not disclose a process for preparing the paroxetine hydrochloride hydrate from a BOC-protected paroxetine as a starting material.

### DISCLOSURE OF INVENTION

The inventors have intensively studied a process for directly preparing a paroxetine hydrochloride hydrate by using the BOC-protected paroxetine. As a result, they have found that the paroxetine hydrochloride hydrate can be efficiently obtained by reacting the BOC-protected paroxetine with hydrogen chloride in the presence of water and thereafter allowing crystals to separate out from the reaction mixture in the presence of water, and they have accomplished the present invention.

Specifically, the present invention relates to:
(1) a process for preparing a paroxetine hydrochloride hydrate, comprising reacting a BOC-protected paroxetine with hydrogen chloride in the presence of water, and allowing crystals to separate out from the resulting reaction mixture in the presence of water;
(2) the process for preparing a paroxetine hydrochloride hydrate according to the item (1) mentioned above, wherein the BOC-protected paroxetine is reacted with hydrogen chloride in the presence of water, and crystals are allowed to separate out from the resulting reaction mixture, without removing the water used in the reaction;
(3) the process for preparing a paroxetine hydrochloride hydrate according to the item (1) or (2) mentioned above, wherein the BOC-protected paroxetine is reacted with hydrogen chloride in the presence of water, and crystals are allowed to separate out from the resulting reaction mixture as they are;
(4) the process for preparing a paroxetine hydrochloride hydrate according to any one of the items (1) to (3) mentioned above, wherein the BOC-protected paroxetine is reacted with hydrogen chloride in an aromatic hydrocarbon-based organic solvent by adding hydrochloric acid to the solvent;
(5) the process for preparing a paroxetine hydrochloride hydrate according to the item (3) mentioned above, wherein the aromatic hydrocarbon-based organic solvent is toluene;
(6) the process for preparing a paroxetine hydrochloride hydrate according to any one of the items (1) to (3) mentioned above, wherein the BOC-protected paroxetine is reacted with hydrogen chloride in a solvent composed only of water;
(7) the process for preparing a paroxetine hydrochloride hydrate according to the item (1), (2), (3) or (6) mentioned above, wherein the BOC-protected paroxetine is reacted with hydrochloric acid;
(8) a paroxetine hydrochloride hydrate prepared by a process of any one of the items (1) to (7) mentioned above; and
(9) a pharmaceutical composition for treating and/or preventing a disorder selected from the group consisting of alcoholism, anxiety syndrome, depression, obsessive-compulsive disorder, panic disorder, chronic pain, obesity, senile dementia, migraine, polyphagia, inappetence, social phobia, premenstrual tension syndrome, adolescent depression, trichotillomania, dysthymia and substance abuse, comprising a paroxetine hydrochloride hydrate prepared by a process of any one of the items (1) to (7) mentioned above, and a pharmaceutically acceptable carrier.

### BEST MODE FOR CARRYING OUT THE INVENTION

In the present invention, the BOC-protected paroxetine is first reacted with hydrogen chloride in the presence of water, to deprotect the tert-butoxycarbonyl (BOC) group which is a protecting group existing at position 1 of the BOC-protected paroxetine (BOC deprotection). The BOC-protected paroxetine may be provided in a solid state such as a crystalline state or an amorphous state, or in an oily state which is prior to a solid state. The BOC-protected paroxetine is preferably a purified one, but can also be a crude one. Also, the BOC-protected paroxetine may be provided in the form of a solution or suspension. Especially preferable is crystalline BOC-protected paroxetine which is purified and has white color or a decolorized color, because the paroxetine gives paroxetine hydrochloride hydrate having a higher purity.

Hydrogen chloride is usually provided in the form of hydrochloric acid which is a solution of hydrogen chloride, in the form of a solution prepared by dissolving hydrogen chloride in a solvent such as a reaction solvent, or in the form of hydrogen chloride gas. The amount of hydrogen chloride is usually sufficient if the amount is at least one mole per mole of the BOC-protected paroxetine, and it is preferable that the amount is usually 1 to 10 moles.

Water may be supplied together with hydrogen chloride as hydrochloric acid, or separately from hydrogen chloride. The water also serves as a reaction solvent as described below. The amount of water used is not particularly limited, and at least one part by weight based on 100 parts by weight of the BOC-protected paroxetine may be sufficient. The amount of water may be the amount of a solvent as described below. It is preferable that the amount of water is usually at most 2000 parts by weight based on 100 parts by weight of the BOC-protected paroxetine.

The reaction of the BOC-protected paroxetine with hydrogen chloride is carried out in a solvent. The solvent is a mixed solvent of water and an organic solvent, or water. A portion or all of the water used in the solvent may be supplied to hydrochloric acid. The proportion of water to the organic solvent in the mixed solvent of water and the organic solvent is not particularly limited, and can be such a low proportion that the amount of water is 1% by weight or so of the organic solvent.

The organic solvent dissolves the BOC-protected paroxetine and hydrogen chloride. The organic solvent is not limited to specified ones, as long as the solvent does not react with the BOC-protected paroxetine and hydrogen chloride. The organic solvent may be a polar solvent which is miscible with water, such as a lower alcohol (methanol, 2-propanol or the like), or a nonpolar solvent which is less miscible with water, such as an aromatic hydrocarbon. A solvent composed only of water or aromatic hydrocarbon-based solvent (for instance, toluene) containing water is preferable, because such solvent can be used in an industrial scale. The organic solvent can be composed of one or more kinds of an organic solvent.

The proportion of the solvent to the BOC-protected paroxetine is not particularly limited. It is preferable that the amount of the solvent is usually 2 to 20 parts by weight or so, based on one part by weight of the BOC-protected paroxetine.

The reaction can be carried out by any means so long as the BOC-protected paroxetine is contacted with hydrogen chloride and the deprotection reaction of BOC group can be achieved, such as a process comprising mixing the BOC-protected paroxetine with hydrogen chloride with stirring in water or a mixture of organic solvent and water, or a process comprising stirring a solution of the BOC-protected paroxetine while introducing hydrogen chloride gas into the solution.

It is preferable that the reaction temperature is usually ordinary temperature to 100°C or so, more preferably 50° to 80°C.

The paroxetine hydrochloride is generated by the hydrochloration of paroxetine which is a deprotected product at the same time that tert-butoxycarbonyl (BOC) which is a protecting group at position 1 of the BOC-protected paroxetine is deprotected.

According to the present invention, a paroxetine hydrochloride hydrate is then generated by allowing crystals to separate out from this paroxetine hydrochloride solution in the presence of water. As a specific method for the crystallization of a paroxetine hydrochloride hydrate, there can be mainly cited a method for allowing the reaction mixture obtained in the above-mentioned deprotection reaction to stand, or a method comprising cooling the reaction mixture to preferably not higher than 30°C, more preferably to 25°C or so, still more preferably to 5°C. At this stage, the reaction mixture can be purified with activated carbon or the like prior to the crystallization. When the crystals are allowed to separate out, water is included in the reaction mixture in an amount of at least 5% by weight, but water may be additionally included in the reaction mixture. When water is sufficiently included in the reaction mixture, an organic solvent may be added to the solution from the viewpoint of the prevention of crystallization of impurities, and the like. Moreover, water, or one or more kinds of organic solvents can be included in the reaction mixture. In this case, the organic solvent may be different from the solvent used in the deprotection reaction of BOC group, as long as the solvent does not interfere with the crystallization. Also, the solvent used in the crystallization may be composed only of water.

When the solvent used in the deprotection reaction of BOC group is mainly composed of an organic nonpolar solvent such as toluene, as another specified method for allowing crystals to separate out from the reaction mixture, there can be cited a method comprising adding water to the reaction mixture obtained after the completion of the deprotection reaction of BOC group, stirring the resulting solution, removing the organic solvent layer from the reaction mixture, and allowing crystals to separate out from the remaining solution containing water. At this stage, the reaction mixture can be purified with activated carbon or the like prior to the crystallization.

When the solvent used in the deprotection reaction of BOC group is an aromatic hydrocarbon such as toluene containing water, the reaction mixture may be separated into three layers, that is, an upper layer which is a layer of an organic solvent such as toluene, an intermediate layer (aqueous layer) and a lower layer (oil layer) when water is added to the solution after the reaction. This lower layer is considered to include water. When crystals are allowed to separate out, the crystallization can be carried out by separating only the lower layer (oil layer) from the solution, and adding a lower alcohol having 1 to 5 carbon atoms, such as 2-propanol to this separated layer to be crystallized. However, from the viewpoint of increasing the yield, it is preferable that both of the intermediate layer (aqueous layer) and the lower layer are separated from the solution, a lower alcohol having 1 to 5 carbon atoms such as 2-propanol is added to these two layers to unite the intermediate layer and the lower layer to one layer, and thereafter crystals of the paroxetine hydrochloride hydrate are allowed to separate out in this one layer. In any of these cases, after the lower alcohol is added, usually, activated carbon or the like is added to the layer in order to be purified, and the layer is warmed or heated to 50° to 85°C or so to completely dissolve the paroxetine hydrochloride. Thereafter, the activated carbon or the like is removed by filtration, and the solution thus obtained is cooled to not higher than 30°C, preferably 25°C or so, more preferably not higher than 5°C, to allow crystals to separate out.

In the present invention, the paroxetine hydrochloride hydrate thus obtained by allowing crystals to separate out is usually separated by a conventional method such as filtration, washed with water and dried.

The present inventors have found that when a warmed or heated solution containing a paroxetine hydrochloride hydrate and water or an organic solvent containing water is cooled to allow crystals to separate out, the crystals usually have a tendency to be colored in pink. However, the paroxetine hydrochloride hydrate being crystallized by the present invention does not suffer from such a problem.

The paroxetine hydrochloride hydrate obtained by the process of the present invention is applied to a medicament for treating and/or preventing a disorder selected from the group consisting of alcoholism, anxiety syndrome, depression, obsessive-compulsive disorder, panic disorder, chronic pain, obesity, senile dementia, migraine, polyphagia, inappetence, social phobia, premenstrual tension syndrome, adolescent depression, trichotillomania, dysthymia and substance abuse.

The pharmaceutical composition of the present invention comprises the above-mentioned paroxetine hydrochloride hydrate and a pharmaceutically acceptable carrier.

The pharmaceutical composition of the present invention comprises the above-mentioned paroxetine hydrochloride hydrate and a carrier. The pharmaceutical composition of the present invention is suitable for treating and/or preventing a disorder selected from the group consisting of alcoholism, anxiety syndrome, depression, obsessive-compulsive disorder, panic disorder, chronic pain, obesity, senile dementia, migraine, polyphagia, inappetence, social phobia, premenstrual tension syndrome, adolescent depression, trichotillomania, dysthymia and substance abuse.

The pharmaceutical composition of the present invention is usually suitable for oral administration, but dissolvable formulation for parenteral administration is also included in the range of the present invention. The pharmaceutical composition of the present invention is usually administrated to a human body in a unit dosage of 1 to 200 mg, preferably 5 to 100 mg, more preferably 10 to 50 mg (for instance, 10, 12.5, 20, 30 or 40 mg) of paroxetine hydrochloride hydrate (active ingredient) on a free base basis. Most preferably, the unit dosage contains 20 mg of paroxetine hydrochloride hydrate (active ingredient) on a free base basis. The composition is usually administrated from once to six times a day, for instance, from twice to four times a day so that the paroxetine hydrochloride hydrate (active ingredient) is administrated in an amount of 5 to 400 mg on a free base basis. More preferably, the composition is administrated in a unit dosage once a day. Preferred form for the dosage of the pharmaceutical composition of the present invention includes a tablet or a capsule. The pharmaceutical composition of the present invention is formulated by a common mixing method such as mixing, filling or compression.

Carriers suitable for the present invention include a diluent, a binder, a disintegrating agent, a colorant, a flavor, a preservative and the like. These carriers can be used alone or in admixture of at least two kinds.

The pharmaceutical composition of the present invention can be used in the same manner as in commercially available antidepressants.

### EXAMPLES

The present invention is more specifically explained on the basis of the following examples, but the scope of the present invention is not limited only to these examples.

### Example 1

To 38.11 g of a toluene solution containing 7.04 g (16.39 mmol) of crude (-)-(3S,4R)-1-tert-butoxycarbonyl-4-(4-fluorophenyl)-3-[(3,4-methylenedioxy)-phenoxymethyl]piperidine was added 4.27 g (equivalent to 41.01 mmol of hydrogen chloride) of 35% hydrochloric acid under nitrogen gas stream, and deprotection of tert-butoxycarbonyl group was carried out at 68° to 70°C for 2 hours. The termination of the reaction was confirmed with high performance liquid chromatography (HPLC). Next, 40 mL of water was added thereto to separate the reaction mixture into three layers at 70°C, and its upper layer (toluene layer) was removed from the reaction mixture, to collect the intermediate layer (aqueous layer) and the lower layer (oil layer). To the intermediate layer and the lower layer was added 10.0 g of 2-propanol to these layers to one layer. In addition, 0.33 g of activated carbon was added thereto, and the mixture was stirred at 62° to 67°C for 15 minutes. Thereafter, the activated carbon was filtered off. In the course of cooling the filtrate to 5°C, crystals were separated out at 28°C. After being stirred at 38° to 40°C for 30 minutes and kept at 2° to 5°C for 30 minutes under ice cooling, the suspension was filtered at the same temperature. The residue was washed with a solution composed of 1.0 g of 2-propanol and 4.0 g of water, to give wet crystals. The crystals were dried at 60°C under reduced pressure, to give 5.10 g of crystals of a paroxetine hydrochloride hydrate (yield: 85.0%). The resulting crystals were confirmed to be an objective product with Nujol infrared absorption (IR) spectrum and X-ray powder diffraction pattern (XRD) by using an authentic sample. The water content was 2.70% by weight (theoretical water content: 2.40% by weight).

### Example 2

To 62.77 g of a toluene solution containing 9.67 g (22.52 mmol) of crude (-)-(3S,4R)-1-tert-butoxycarbonyl-4-(4-fluorophenyl)-3-[(3,4-methylenedioxy)-phenoxymethyl]piperidine was added 5.86 g (equivalent to 56.30 mmol of hydrogen chloride) of 35% hydrochloric acid under nitrogen gas stream, and deprotection of tert-butoxycarbonyl group was carried out at 70° to 73°C for 2 hours. The termination of the reaction was confirmed with high performance liquid chromatography (HPLC). Next, 35 mL of water was added thereto, to separate the reaction mixture into three layers at 70°C, and its upper layer (toluene layer) and its intermediate layer (aqueous layer) were removed from the reaction mixture, to collect the lower layer (oil layer). To this lower layer was added 67.69 g of 2-propanol, and 1.08 g of activated carbon was added thereto. The mixture was stirred at 81°C for 15 minutes, and thereafter the activated carbon was filtered off. The filter cake was washed with 4.74 g of 2-propanol being previously warmed. In the course of cooling the resulting filtrate to 5°C, crystals were crystallized at 30°C. After being stirred at 25° to 30°C for 30 minutes and kept at 3° to 5°C for 30 minutes under ice cooling, the suspension was filtered at the same temperature. The residue was washed with 19.41 g of 2-propanol, to give wet crystals. The crystals were dried under reduced pressure at 60°C, to give 4.68 g of crystals of a paroxetine hydrochloride hydrate (yield: 55.5% by weight). The crystals were confirmed to be an objective product with Nujol infrared absorption (IR) spectrum and X-ray powder diffraction pattern (XRD) by using an authentic sample. The water content was 2.42% by weight (theoretical water content: 2.40% by weight).

### Example 3

### 1) Purification of crude (-)-(3S, 4R)-1-tert-butoxycarbonyl-4-(4-fluorophenyl)-3-[(3, 4-methylenedioxyphenyl)oxymethyl]piperidine

To 71.50 g (166.5 mmol) of crude (-)-(3S,4R)-1-tert-butoxycarbonyl-4-(4-fluorophenyl)-3-[(3,4-methylenedioxyphenyl)oxymethyl]piperidine was added 214 mL of 2-propanol, and the mixture was heated to 58°C. When the crude compound was completely dissolved, 0.70 g of activated carbon was added thereto, and the mixture was stirred with heating under reflux for 10 minutes.

The activated carbon was filtered off at the same temperature and the filter cake was washed with 36 mL of 2-propanol. The filtrate was cooled to 2°C. After the filtrate was allowed with stirring at 2° to 8°C under ice cooling for 2 hours and 20 minutes, the suspension was filtered at the same temperature. The residue was washed with 36 mL of 2-propanol, to give crystals. The crystals were dried under reduced pressure at 50°C, to give 60.83 g of purified (-)-(3S, 4R)-1-tert-butoxycarbonyl-4-(4-fluorophenyl)-3-[(3,4-methylenedioxyphenyl)-oxymethyl]piperidine (yield: 85.1% by weight).

### 2) Preparation of a paroxetine hydrochloride hydrate

Next, 5.87 g (13.67 mmol) of the purified (-)-(3S,4R)-1-tert-butoxycarbonyl-4-(4-fluorophenyl)-3-[(3,4-methylenedioxyphenyl)oxymethyl]-piperidine was dissolved in 34 mL of toluene, and 7.12 g (68.36 mmol) of 35% hydrochloric acid was added dropwise thereto. The deprotection reaction of BOC group was carried out at 67° to 72°C for 7 hours, and the termination of the reaction was confirmed with HPLC.

Thereafter, the mixture was cooled as it is and allowed with stirring at 21° to 25°C for 30 minutes to allow the crystals to crystallize. The crystals were then filtered off at the same temperature. The crystals were washed with 6.8 mL of toluene, to give 4.85 g (94.7% by weight) of crystals of a paroxetine hydrochloride hydrate. It was confirmed that the crystal was an objective product with Nujol IR spectrum and powdered X-ray diffraction pattern (XRD). The water content was 2.69% by weight (theoretical water content: 2.40% by weight).

### Example 4

In 27.0 mL of water was suspended 5.87 g (13.67 mmol) of the purified (-)-(3S,4R)-1-tert-butoxycarbonyl-4-(4-fluorophenyl)-3-[(3,4-methylenedioxyphenyl)oxymethyl]piperidine under nitrogen gas stream, and 7.12 g (68.36 mmol) of 35% hydrochloric acid was added dropwise thereto. The deprotection reaction of BOC group was carried out under reflux for 1 hour, and the termination of the reaction was confirmed with HPLC.

After the termination of the reaction, the resulting reaction mixture was cooled to 5°C. In the course of cooling, crystals were separated out at 58°C. After being kept at 2° to 5°C under ice cooling for 30 minutes, the mixture was filtered at the same temperature. The residue was washed with 5.87 g of water, to give wet crystals. The crystals were dried under reduced pressure at 60°C, to give 4.90 g of crystals of a paroxetine hydrochloride hydrate (yield: 95.7% by weight). The water content was 2.53% by weight (theoretical water content: 2.40% by weight).

It can be seen from the above results that according to the present invention, a paroxetine hydrochloride hydrate is successfully obtained from a BOC-protected paroxetine.

### INDUSTRIAL APPLICABILITY

The paroxetine hydrochloride hydrate of the present invention can be suitably used as an antidepressant for treating and/or preventing a disorder selected from the group consisting of alcoholism, anxiety syndrome, depression, obsessive-compulsive disorder, panic disorder, chronic pain, obesity, senile dementia, migraine, polyphagia, inappetence, social phobia, premenstrual tension syndrome, adolescent depression, trichotillomania, dysthymia and substance abuse.

## Claims

1. A process for preparing a paroxetine hydrochloride hydrate, comprising reacting (3S, 4R)-1-tert-butoxycarbonyl-4-(4-fluorophenyl)-3-[(3,4-methylenedioxy)phenoxymethyl]piperidine with hydrogen chloride in the presence of water, and allowing crystals to separate out from the resulting reaction mixture in the presence of water.

2. The process for preparing a paroxetine hydrochloride hydrate according to claim 1, wherein the (3S, 4R)-1-tert-butoxycarbonyl-4-(4-fluorophenyl)-3-[(3,4-methylenedioxy)phenoxymethyl]piperidine is reacted with hydrogen chloride in the presence of water, and crystals are allowed to separate out from the resulting reaction mixture, without removing the water used in the reaction.

3. The process for preparing a paroxetine hydrochloride hydrate according to claim 1 or 2, wherein the (3S, 4R)-1-tert-butoxycarbonyl-4-(4-fluorophenyl)-3-[(3,4-methylenedioxy)phenoxymethyl]piperidine is reacted with hydrogen chloride in the presence of water, and allowing crystals to separate out from the resulting reaction mixture as they are.

4. The process for preparing a paroxetine hydrochloride hydrate according to any one of claims 1 to 3, wherein the (3S, 4R)-1-tert-butoxycarbonyl-4-(4-fluorophenyl)-3-[(3,4-methylenedioxy)phenoxymethyl]piperidine is reacted with hydrogen chloride in an aromatic hydrocarbon-based organic solvent by adding hydrochloric acid to the solvent.

5. The process for preparing a paroxetine hydrochloride hydrate according to claim 4, wherein the aromatic hydrocarbon-based organic solvent is toluene.

6. The process for preparing a paroxetine hydrochloride hydrate according to any one of claims 1 to 3, wherein the (3S, 4R)-1-tert-butoxycarbonyl-4-(4-fluorophenyl)-3-[(3,4-methylenedioxy)phenoxymethyl]piperidine is reacted with hydrogen chloride in a solvent composed only of water.

7. The process for preparing a paroxetine hydrochloride hydrate according to claim 1, 2, 3 or 6, wherein the (3S, 4R)-1-tert-butoxycarbonyl-4-(4-fluorophenyl)-3-[(3,4-methylenedioxy)phenoxymethyl]piperidine is reacted with hydrochloric acid.

8. A paroxetine hydrochloride hydrate prepared by a process of any one of claims 1 to 7.

9. A pharmaceutical composition for treating and/or preventing a disorder selected from the group consisting of alcoholism, anxiety syndrome, depression, obsessive-compulsive disorder, panic disorder, chronic pain, obesity, senile dementia, migraine, polyphagia, inappetence, social phobia, premenstrual tension syndrome, adolescent depression, trichotillomania, dysthymia and substance abuse, comprising a paroxetine hydrochloride hydrate prepared by a process of any one of claims 1 to 7, and a pharmaceutically acceptable carrier.
